(19) Europäisches Patentamt
European Patent Office
Office européen des brevets

(11) Veröffentlichungsnummer: **0 029 116**
**B1**

(12) **EUROPÄISCHE PATENTSCHRIFT**

(45) Veröffentlichungstag der Patentschrift: **05.09.84**

(21) Anmeldenummer: **80106151.6**

(22) Anmeldetag: **10.10.80**

(51) Int. Cl.³: **C 07 D 471/22,** A 61 K 31/475 // (C07D471/22, 221/00, 221/00, 221/00, 209/00)

(54) **N(b)-quartäre Derivate von 10-Bromsandwicin und 10-Bromisosandwicin, Verfahren und Zwischenprodukte zur Herstellung der Derivate und diese Derivate enthaltende Arzneimittel.**

(30) Priorität: **13.10.79 DE 2941530**

(43) Veröffentlichungstag der Anmeldung: **27.05.81 Patentblatt 81/21**

(45) Bekanntmachung des Hinweises auf die Patenterteilung: **05.09.84 Patentblatt 84/36**

(84) Benannte Vertragsstaaten: **AT BE CH DE FR GB IT LI LU NL SE**

(56) Entgegenhaltungen:
**DE-A-2 611 162**
**DE-A-2 701 417**
**FR-A-2 392 995**

**Die Akte enthält technische Angaben, die nach dem Eingang der Anmeldung eingereicht wurden und die nicht in dieser Patentschrift enthalten sind.**

(73) Patentinhaber: **Kali-Chemie Pharma GmbH**
**Hans-Böckler-Allee 20 Postfach 220**
**D-3000 Hannover 1 (DE)**

(72) Erfinder: **Kehrbach, Wolfgang Dipl.-Chem., Dr.rer.nat.**
**Altenbekener Damm 41**
**D-3000 Hannover 1 (DE)**
Erfinder: **Wegener, Joachim Dipl.-Chem., Dr.rer.nat.**
**Mühlenstrasse 41**
**D-3201 Algermissen (DE)**
Erfinder: **Kühl, Ulrich, Dr.med.vet.**
**Bergener Strasse 9**
**D-3000 Hannover 61 (DE)**
Erfinder: **Budden, Renke, Dr.med.vet.**
**Rodewaldstrasse 18**
**D-3000 Hannover (DE)**
Erfinder: **Buschmann, Gerd Dr.med.vet**
**Matthäikirchstrasse 27**
**D-3000 Hannover 81 (DE)**

(74) Vertreter: **Lauer, Dieter, Dr.**
**c/o Kali-Chemie Aktiengesellschaft Postfach 220**
**D-3000 Hannover 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 029 116 B1

Courier Press, Leamington Spa, England.

**Beschreibung**

Die Erfindung betrifft $N_b$-quartäre Derivate von 10-Bromsandwicin und 10-Bromisosandwicin, Verfahren und Zwischenprodukte zur Herstellung der Derivate und die Derivate enthaltende Arzneimittel.

Es ist bekannt (DE—A—26 11 162, DE—A—27 01 417), daß sich $N_b$-quartäre Derivate von Sandwicin und Isosandwicin durch wertvolle pharmakologische, insebesondere antiarrhythmische Wirkungen auszeichnen.

Bekannt sind auch die zur Sandwicin- bzw. Isosandwicin-Reihe epimeren $N_b$-quartären Derivate von Ajmalin und Isoajmalin (DE—C—11 54 120, DE—C—11 96 207, DE—C—16 20 559, DE—A—20 25 286). Zwei Vertreter aus der letztgenannten Gruppe der $N_b$-quartären Ajmalin- bzw. Isoajmalin-Derivate sind Wirkstoffe von im Handel befindlichen Präparaten, nämlich das $N_b$-Propyl-ajmalinium Hydrogentartrat im Präparat Neo-Gilurytmal[R] und das $N_b$-(2-Hydroxy-3-diäthylamino-)propyl-ajmalinium Hydrogentartrat im Präparat Tachmalcor[R].

Bekannt ist schließlich auch das 10-Bromajmalin (Anet et al., J. Chem. Soc, 1954, Part I, 1242), während das 10-Bromsandwicin nicht literaturbekannt ist. Aus FR—A—2392995 ist die Synthese quartärer Sandwicine über die entsprechenden Aldehydbasen bekannt.

Der Erfindung liegt die Aufgabe zugrunde, bessere Wirkstoffe als die bekannten $N_b$-quartären Sandwicin- und Isosandwicin-bzw. Ajmalin- und Isoajmalin-Derivate bereitzustellen.

Die Lösung dieser Aufgabe gelingt durch Bereitstellung von $N_b$-quartären 10-Bromsandwicin und 10-Bromisosandwicin-Derivaten der allgemeinen Formel I

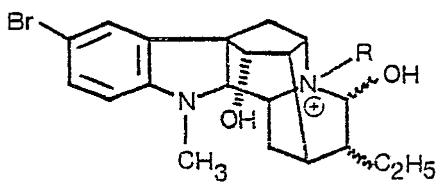

$A^{\ominus}$ (I)

in der

A das Anion einer anorganischen oder organischen Säure und

R ein bis zu 10 Kohlenstoffatome und gegebenenfalls Halogen oder Sauerstoff oder Stickstoff oder Sauerstoff und Stickstoff enthaltender Rest ist,

wobei

a) R ein Rest der allgemeinen Formel IIa

$$-(CH_2)_n-\underset{\displaystyle Y}{\underset{|}{CH}}-CH_2-X \qquad \text{(IIa)}$$

oder

b) R ein Rest der allgemeinen Formel IIb

$$-CH_2-\underset{\displaystyle OH}{\underset{|}{CH}}-CH_2-X \qquad \text{(IIb)}$$

ist, in denen

n den Wert 0 oder 1 annehmen kann

X Wasserstoff,
gerad- oder verzweigtkettiges Alkyl,
gegebenenfalls substituiertes Phenyl,
Hydroxy,
Dialkylamino,
Pyrrolidino,
Piperidino oder
Morpholino und

Y Wasserstoff oder Methyl bedeuten oder

c) R ein Methyl-,
Allyl-,
Benzyl-,
4-Fluorbenzyl-,
4-Methoxybenzyl- oder
2-Hydroxy-2-phenyl-äthyl-Rest

ist.

Der Rest R ist insbesondere ein Methyl-, Äthyl-, Allyl-, Propyl-, Butyl-, 3-Methyl-butyl-, Hexyl-,

Decyl-2-Hydroxy-äthyl-, 2-Diäthylamino-äthyl-, 3-Diäthylaminopropyl-, 2-(1-Pyrrolidinyl)-äthyl-, 2-(1-Piperidinyl)-äthyl-, 2-(4-Morpholinyl)-äthyl- oder 2-Hydroxy-3-(1-piperidinyl)-propyl-Rest.

Vorteilhafterweise ist A das Anion einer pharmakologisch annehmbaren Säure, vorzugsweise das Anion der Weinsäure, Oxalsäure, Citronensäure, Salzsäure oder Phosphorsäure, insbesondere das Anion der Weinsäure.

Die neuen Derivate können auf verschiedene Weise hergestellt werden.

In einem Fall wird 10-Bromsandwicin der Formel III-n

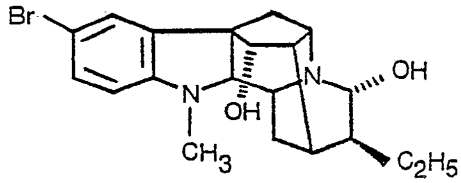

(III-n)

in der die 21-Hydroxy-Gruppe $\alpha$-ständig und die 20-Äthyl-Gruppe $\beta$-ständig ist, oder 10-Bromisosandwicin der Formel III-i

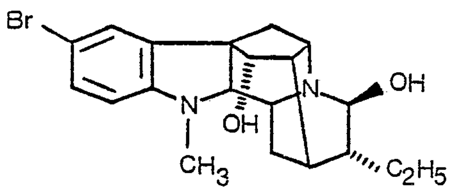

(III-i)

in der die 21-Hydroxy-Gruppe $\beta$-ständig und die 20-Äthyl-Gruppe $\alpha$-ständig ist, entweder mit einer bis zu 10 Kohlenstoffatome enthaltenden Verbindung der Formeln Va oder Vb

$$\begin{array}{l} Z-(CH_2)_n-CH-CH_2-X \\ \qquad\qquad\quad\; | \\ \qquad\qquad\quad\; Y \end{array} \qquad (Va)$$

$$\begin{array}{l} Z-CH_2-CH-CH_2-X \\ \qquad\qquad\; | \\ \qquad\qquad\; OH \end{array} \qquad (Vb)$$

in denen, n, X und Y die oben genannte Bedeutung haben und Z Chlor, Brom, Jod oder die Tosylgruppe bedeutet, oder mit

Methyl-,
Allyl-,
Benzyl-,
4-Fluorbenzyl-,
4-Methoxybenzyl-Chlorid, Bromid, Jodid, oder Tosylat

umgesetzt.

Die Ausgangsstoffe können dabei in äquimolaren Mengen eingesetzt werden. Vorteilhafterweise wird ein Überschuß des "Alkylierungsmittels" verwendet. Die Umsetzung erfolgt vorzugsweise in einem Lösungsmittel, das gegenüber den Reaktionsteilnehmern inert ist. Geeignete Lösungsmittel sind beispielweise Acetonitril, Chloroform, Dimethylformamid, Sulfolan, Dioxan oder Alkohole, wie z.B. Methanol oder Äthanol. Soweit das "Alkylierungsmittel" unter den Reaktionsbedingungen nicht mit sich selbst reagiert, kann auch das "Alkylierungsmittel" als Lösungsmittel dienen. Vorteilhafterweise führt man die Umsetzung bei der Siedetemperatur des Lösungsmittels aus, die Umsetzung kann aber insbesondere bei den höher siedenden Lösungsmitteln auch unterhalb der Siedetemperatur ausgeführt werden.

Die aus der vorerwähnten Umsetzung resultierenden quartären Salze von 10-Bromsandwicin- oder 10-Bromisosandwicin können bereits Endprodukte im Sinne der Formel I sein, beispielweise wenn das quartäre Salz für die beabsichtigte gelanische Zubereitung geeignet oder das Anion des quartären Salzes ein pharmakologisch annehmbares Anion ist.

Wenn das quartäre Salz für die beabsichtigte Verwendung nicht geeignet ist, beispielsweise weil das Salz hygroskopisch oder das Anion pharmakologisch nicht annehmbar ist, werden die erhaltenen quartären Salze durch Behandlung mit Alkalien in die ringoffenen Aldehydbasen der Formel IV

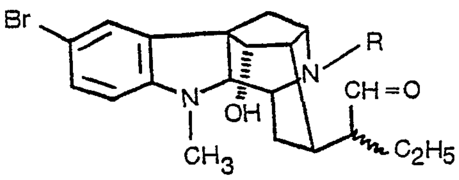

(IV)

in der R die vorstehend genannte Bedeutung hat, umgewandelt und diese durch Umsetzung mit einer Säure HA, in der A die vorstehend genannte Bedeutung hat, in das quartäre Derivat der Formel I überführt.

Als zur Umwandlung der quartären Salze in die ringoffene Aldehydbase geeignete Alkalien kommen wässrige Laugen, wie z.B. 10%-ige Natronlauge oder wässrige Natriumhydrogencarbonat- oder Natriumcarbonat-Lösungen in Betracht. Zweckmäßigerweise wird in Gegenwart eines geeigneten Extraktionsmittels gearbeitet. Geeignet sind alle inerten wassernichtmischbaren Lösungsmittel, wie Chloroform, Methylenchlorid, Essigester oder Diäthyläther, in denen die Basen eine ausreichende Löslichkeit besitzen. Nach Abdestillation des Extraktionsmittels, vorteilhafterweise Vakuumdestillation, liegen die Basen in amorpher Form vor. Für die Weiterverarbeitung in das quartäre Derivat ist es nicht unbedingt erforderlich, die ringoffene Aldehydbase zu isolieren; es kann auch der getrocknete und geklärte Extrakt verwendet werden.

In einer anderen Verfahrensvariante wird 10-Bromsandwicin oder 10-Bromisosandwicin mit einem Epoxid der Formel Vb′

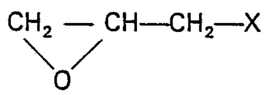

(Vb′)

in der X die oben genannte Bedeutung hat oder mit Epoxystyrol umgesetzt.

Im Fall der Umsetzung von 10-Bromsandwicin oder 10-Bromisosandwicin mit Epoxiden fällt sofort die ringoffene Aldehydbase an, die man mit der Säure HA, in der A die vorstehend genannte Bedeutung hat, in das quartäre Derivat der Formel I überführt.

Da die ringoffenen Aldehydbasen in amorpher, d.h. nicht kristalliner Form vorliegen, besitzen sie keinen eindeutigen Schmelzpunkt. Auch der Drehwert scheidet für eine eindeutige Charakterisierung aus, da bei der Quarternisierung von Bromsandwicin bzw. Bromisosandwicin eine Isomerisierung an den Zentren C-20 und C-21 auftreten kann. Diese Isomerisierung führt, gleichgültig ob man von reinem Bromsandwicin oder reinem Bromisosandwicin ausgeht, zu einem Gemisch der stereoisomeren quartären Salze bzw. Aldehydbasen, wobei die Zusammensetzung des Gemiches jedoch unterschiedlich ist, je nach dem, ob man von Bromsandwicin oder Bromisosandwicin ausgeht. Der Grad der Isomerisierung ist vom Raumanspruch des Restes R und der Art seiner Substitution abhängig. Das Isomerenverhältnis kann auch von Versuch zu Versuch schwanken, wenn die Reaktionsbedingungen nicht exakt übereinstimmen. Bei der Überführung der ringoffenen Aldehydbasen in die quartären Derivate kann sich das Verhältnis der Isomeren weiter verändern.

Die ringoffenen Aldehydbasen lassen sich daher am vorteilhaftesten durch die Lage der Resonanz des aldehydischen Protons im $^1$H-Kernresonanzspektrum und durch das Auftreten einer Carbonyl-Bande im IR-Spektrum charakterisieren. Die Resonanz des aldehydischen Protons tritt bei 9,0—9,6 auf, während die Carbonylbande bei 1700—1720 cm$^{-1}$ erscheint. Als repräsentativ für die ringoffenen Aldehydbasen ist in Fig. 1 das IR-Spektrum der ringoffenen Aldehydbase von $N_b$-Hexyl-10-bromisosandwicin und in Fig. 2 ein Aussschnitt aus dem 90 MHz $^1$-H-FT-NMR-Spektrum derselben Verbindung dargestellt. Im IR-Spektrum ist die C=O Valenzsschwingung bei 1710 cm$^{-1}$ zu erkennen. Im NMR-Spektrum ist die bei tiefem Feld auftretende Resonanz des O=CH-Aldehydprotons am C 21 charakteristisch, die durch Kopplung mit dem einzigen benachbarten Proton am C 20 mit einer Frequenz von 4 Hz aufgespalten ist. Das Auftreten von 2 Signalen ist auf das Vorliegen eines Isomerengemisches aus der n-Form (Signal bei 9,50) und der iso-Form (Signal bei 9,43) zurückzuführen. Aus der Höhe der jeweiligen Peaks kann auf das Isomeren-Verhältnis geschlossen werden.

Die in Substanz dargestellten oder im Extraktionsmittel gelöst vorliegenden ringoffenen Aldehydbasen werden anschließend mit einer pharmakologisch annehmbaren anorganischen oder organischen Säure umgesetzt, vorzugsweise mit Weinsäure, Oxalsäure, Citronensäure, Salzsäure oder Phosphorsäure und dabei in die quartären Derivate überführt.

Soweit vorstehend oder nachfolgend von n- oder iso-Form gesprochen wird, bezieht sich diese Aussage ausschließlich auf die Konfiguration am C-20. Die 21-OH-Gruppe steht üblicherweise transständig, kann aber auch cis-ständig sein.

Die erfindungsgemäßen $N_b$-quartären Derivate von 10-Bromsandwicin bzw. 10-Bromisosandwicin zeigen bei arzneilicher Verwendung adrenolytische und herzrhythmisierende Wirkungen. Überraschenderweise sind jedoch die neuen Verbindungen wesentlich stärker wirksam und physiologisch verträglicher als die bekannten Ajmalin-Derivate. So zeigen die neuen Derivate im Vergleich zu den bekannten Ajmalin-Derivaten schon bei einer wesentlich geringeren Dosis ein

vergleichbare Wirkung. Darüberhinaus ist die unerwünschte negative Inotropie, wie sie bei den Ajmalin-Derivaten beobachtet wird, schwächer, und zwar bei gleichzeitiger größerer therapeutischer Breite.

Die erfindungsgemäß hergestellten Wirkstoffe sind sowohl in wäßriger Lösung als auch in fester form stabil und haltbar und können mit den üblichen Träger- oder Hilfsstoffen in arzneilich geeigneten Darreichungsformen therapeutisch angewendet werden.

Die Überlegenheit der erfindungsgemäßen Wirkstoffe ergibt sich aus dem Vergleich der in der nachstehenden Tabelle für repräsentative Vertreter angegebenen pharmakologischen Daten mit den entsprechenden Daten des bekannten $N_b$-Propyl-Ajmalinium-Hydrogentartrat (Neo-Gilurytmal®).

In der Tabelle ist im einzelnen aufgeführt:

Die aktute Toxizität bei oraler (p.o.) und intraperitonealer (i.p.) Applikation an männlichen NMRI-Mäusen der Gewichtsklasse 18 bis 22 g. Als $LD_{50}$ ist dabei diejenige Dosis in $\mu$mol/kg definiert, bei der die Mortalitätsrate am 7. Tag nach Applikation 50% der Versuchstiere beträgt. Die $LD_{50}$ wurde durch Probitanalyse [L. Cavalli-Sforza, Grundbegriffe der Biometrie, insbesondere der statistischen Methoden bei der Wertbemessung biologisch wirksamer Substanzen, Gustav Fischer Verlag, Stuttgart (1964)] bereichnet.

Minimale Symptomdosis bei männlichen NMRI-Mäusen der Gewichtsklasse 18 bis 22 g gemäß dem Wirkungsbild nach Campbell und Richter [D. E. S. Campbell und W. Richter, Acta Pharmakol. Toxicol. *25* (1967), 345—363]: Als minimale Symptomdosis ist diejenige Dosis in $\mu$mol/kg definiert, bei welcher es bei 2 von 3 Mäusen nach i.p. Verabreichung zu Verhaltersänderungen kam. Die minimale Symptomdosis ist ein Maß für die unerwünschten Nebenwirkungen.

Verlängerung der funktionellen Refraktärzeit (fRZ.) und die Verminderung der Kontraktionskraft (Kraft) am isolierten linken Vorhof von weiblichen Albino-Pirbright-white-Meerschweinchen (MS) der Gewichtsklasse 300 bis 400 g gemäß der Doppelreizmethode von Govier [W. C. Govier, J. Pharmakol. Exp. Ther. *148* (1) (1965), 100—105]: Angegeben ist diejenige Konzentration in $\mu$mol/l, bei der es 18 Minuten nach Applikation zu einer Verlängerung der funktionellen Refraktärzeit auf 125% bzw. zu einer Verminderung der Kontraktionskraft auf 75% des Ausgangswertes kommt. Angegeben ist außerdem der Quotient aus kontraktionskraftmindernder und refraktärzeitverlängernder Dosis. Dieser Quotient gibt Aufschluß über die "therapeutische Breite" der antiarrhythmischen Wirkung am isolierten Organ [K. Greef, Verh. Dtsch. Ges. Kreislaufforsch. *35* (1969) 88—97].

Da einige der Daten in der Dimension $\mu$mol/kg bzw. $\mu$mol/l angegeben sind, ist in der Tabelle außerdem noch das errechnete Molekulargewicht (MG) der Wirksubstanz angegeben.

TABELLE

| Beispiel | Wirksubstanz R | MG | $LD_{50}$ i.p. µmol/kg | $LD_{50}$ p.o. µmol/kg | Min. Symptom-dosis µmol/kg | isolierter MS–Vorhof Kraft µmol/l | isolierter MS–Vorhof fRZ µmol/l | isolierter MS–Vorhof Kraft/fRZ |
|---|---|---|---|---|---|---|---|---|
| Vergleich (Neo-Gilurytmal) | n-$C_3H_7$ | 519 | 41 | 65 | 12 | 0,26 | 1,8 | 0,15 |
| 1.b) | $-CH_3$ | 569 | 325 | 1910 | 11 | 20,2 | 3,2 | 6,4 |
| 2.b) | n-$C_3H_7$ | 598 | 101 | 1133 | 42 | 4,7 | 1,4 | 3,3 |
| 4.b) | n-$C_6H_{13}$ | 640 | 112 | >2300 | 39 | 2,0 | 4,3 | 0,5 |
| 8) | $-CH_2-CHOH-CH_2-$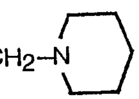 | 847 | 169 | 1190 | 59 | 3,4 | 8,3 | 0,4 |
| 12.b) | n-$C_3H_7$ | 598 | 118 | 566 | 10 | 6,5 | 1,8 | 3,6 |

Die Herstellung des als Ausgangsverbindungen benötigten 10-Bromsandwicin und 10-Bromisosandwicin erfolgte nach folgenden Vorschriften:

Arbeitsvorschrift für die Darstellung von 10-Bromsandwicin

26,6 g Sandwicin wurden in 2 Litern eines Gemischs aus Tetrahydrofuran und Methylenchlorid (4:1; V:V) gelöst und bei −5°C unter Rühren portionsweise mit 33 g 2,4,4,6-Tetrabrom-2,5-cyclohexadien-1-on versetzt. Nach der letzten Zugabe wurde noch 30 Minuten bei −5°C bis −10°C gerührt, auf Raumtemperatur erwärmt, mit weiterem Methylenchlorid versetzt, zweimal mit 2n Natronlauge und anschließend zweimal mit Wasser gewaschen, die organische Phase eingeengt, in 500 ml Methanol aufgenommen und zur methanolischen Lösung langsam Wasser zugetropft, bis die Fällung des 10-Bromsandwicins vollständig war. Das Produkt wurde abgesaugt, mit Wasser, dann mit kaltem Aceton gewaschen und getrocknet.

Ausbeute: 30,1 g (91%)
Fp.: 204°C
reine n-Form

Durch Ansäuern der wäßrig alkalischen Extrakte konnte 2,4,6-Tribromphenol gewonnen werden, das nach Umkristallisation aus Petroläther erneut zur Darstellung von, 2,4,4,6-Tetrabrom-2,5-cyclohexadien-1-on verwendet wurde.

Arbeitsvorschrift für die Darstellung von 10-Bromisosandwicin

15 g 10-Bromsandwicin und 20 g Kaliumhydroxid wurden in 700 ml Methanol gelöst und 8 Stunden unter Rückfluß erhitzt. Nach dem Verdünnen mit 400 ml Wasser wurde dreimal mit Methylenchlorid extrahiert, getrocknet, eingeengt und das Produkt aus Methanol kristallisiert.

Ausbeute: 10,5 g (70%)
Fp.: 173—175°C
reine iso-Form

In der Mutterlauge verbleibt ein Gemisch aus 10-Bromsandwicin und wenig 10-Bromisosandwicin, das erneut isomerisiert werden kann.

Die folgenden Beispiele sollen den Gegenstand der Erfindung näher erläutern.

Die aus 10-Bromsandwicin bzw. 10-Bromisosandwicin gemäß den Beispielen hergestellten quartären Salze wurden nach Freisetzung der Aldehydbase mit L(+)-Weinsäure zu den Hydrogentartraten umgesetzt. Aldehydbasen mit stickstofffreien Alkylrest wurden mit einer äquimolaren Weinsäuremenge, Aldehydbasen mit stickstoffhaltigem Alkylrest mit einer doppelt molaren Weinsäuremenge umgesetzt.

Beispiele 1—10

Alkylierung von 10-Bromsandwicin

1a) $N_b$-Methyl-10-bromsandwiciniumjodid

12 g 10-Bromsandwicin und 13 ml Methyljodid wurden in 500 ml Acetonitril 8 Stunden unter Rückfluß erhitzt. Der ausgefallene Niederschlag wurde abfiltriert und mit Essigester gewaschen.

Ausbeute: 9,5 g (59%)
Fp.: 240—242°C
reine n-Form

1b) $N_b$-Methyl-10-bromsandwicinium-hydrogentartrat

8,7 g $N_b$-Methyl-10-bromsandwiciniumjodid wurden mit verdünnter Sodalösung aufgenommen und mit Essigester extrahiert. Zu dieser Lösung tropfte man eine Lösung von 2,4 g L(+)-Weinsäure in Aceton, filtrierte den ausgefallenen Niederschlag ab und wusch mit Essigester.

Ausbeute: 8,0 g (52%)
Fp.: 166—170°C
reine n-Form

2a) $N_b$-n-Propyl-10-bromsandwiciniumjodid

Die Alkylierung mit n-Propyljodid erfolgte analog Beispiel 1a.

Ausbeute: 67%
Fp.: 270°C (dec)
reine n-Form

2b) $N_b$-n-Propyl-10-bromsandwicinium-hydrogentartrat

Die Umwandlung ins Hydrogentartrat erfolgte analog Beispiel 1b.

Ausbeute: 57%
Fp.: 153—155°C
reine n-Form

3) $N_b$-Allyl-10-bromsandwicinium-bromid

1 g 10-Bromsandwicin und 1 ml Allylbromid wurden in 30 ml Acetonitril gelöst und 8 Stunden

unter Rückfluß erhitzt. Das ausgefallene $N_b$-Allyl-10-bromsandwiciniumbromid wurde abgesaugt, mit Aceton gewaschen und getrocknet.

Ausbeute: 1,3 g (100%)
Fp.: 263°C (dec)

4a) $N_b$-n-Hexyl-10-bromsandwiciniumjodid
Die Alkylierung mit n-Hexyljodid erfolgte analog Beispiel 1a.
Ausbeute: 67%
Fp.: 224—227°C
reine n-Form

4b) $N_b$-n-Hexyl-10-bromsandwicinium-hydrogentartrat
Die Umwandlung ins Hydrogentartrat erfolgte analog Beispiel 1b.
Ausbeute: 38%
Fp.: 134°C
reine n-Form

5) $N_b$-Decyl-10-bromisosandwicinium-bromid

1 g 10-Bromsandwicin und 1 ml 1-Bromdecan wurden in 15 ml Äthanol gelöst und 48 g unter Rückfluß erhitzt. Das ausgefallene $N_b$-Decyl-10-bromsandwicinium-bromid wurde abgesaugt, mit kaltem Äthanol gewaschen und getrocknet.

Ausbeute: 0,6 g (39%)
Fp.: 258°C
n: iso = 3:7

6) $N_b$-(3-methylbutyl)-bromsandwicinium-jodid

1 g 10-Bromsandwicin und 1 ml 1-Jod-3-methylbutan wurden in 15 ml Äthanol gelöst und 24 h unter Rückfluß erhitzt. Nach dem Abkühlen wurde mit Äther versetzt und das ausgefallene $N_b$-(3-Methylbutyl)-10-bromsandwicinium-jodid abfiltriert, mit Äther gewaschen und getrocknet.

Ausbeute: 0,5 g (34%)
Fp.: 266°C (dec)

7) $N_b$-Benzyl-10-bromsandwicinium-bromid

1 g 10-Bromsandwicin und 1 ml Benzylbromid wurden in 35 ml Acetonitril gelöst und 8 h unter Rückfluß erhitzt. Das ausgefallene $N_b$-Benzyl-10-bromsandwiciniumbromid wurde abfiltriert, mit kaltem Aceton gewaschen und getrocknet.

Ausbeute: 1 g (70%)
Fp.: 242°C
n: Iso = 6:4

8) $N_b$-[2-hydroxy-3-(1-piperidinyl)-propyl]-10-bromsandwicinium-bishydrogentartrat

10 g 10-Bromsandwicin und 3,7 g 3-Piperidino-1,2-epoxypropan wurden in 75 ml Äthanol gelöst und bei 75°C gerührt. Nach 8 h fügte man nochmals 1 g 3-Piperidino-1,2-epoxypropan zu und rührte weitere 8 h bei 75°C. Man engte im Vakuum zur Trockene ein, nahm den Rückstand in ca. 100 ml Aceton auf und tropfte die erhaltene Lösung unter Rühren zu einer mit Eis gekühlten Lösung von 3,9 g L(+)-Weinsäure in 250 ml Aceton. Das ausgefallene Bishydrogentartrat wurde abfiltriert und mit Aceton und Äther nachgewaschen. Aus dem Bishydrogentartrat wurde mit Sodalösung die Aldehydbase freigestezt, mit Äther extrahiert und erneut mit L(+)-Weinsäure das Bishydrogentartrat gefällt.

Ausbeute: 9,4 g (45%)
Fp.: 135°C

9) $N_b$-(2-hydroxy-2-phenyläthyl)-10-bromsandwicinium-hydrogentartrat

1 g 10-Bromsandwicin und 0,4 ml Epoxystyrol wurden in 15 ml Äthanol gelöst und 7 h auf 75°C erhitzt. Nach Zugabe von weiteren 0,3 ml Epoxystyrol wurde noch 8 h auf 75°C erhitzt, eingeengt, mit Methanol aufgenommen und nach Zugabe von 0,32 g L(+)-Weinsäure langsam in 80 ml Essigester eingetropft. Das ausgefallene Hydrogentartrat wurde abgesaugt, mit Essigester gewaschen und getrocknet.

Ausbeute: 1,5 g (90%)
Fp.: 139°C

10) $N_b$-(2-Hydroxyäthyl)-10-bromoisosandwicinium-hydrogentartrat

2 g 10-Bromsandwicin und 7 ml Chloräthanol wurden in 10 ml Sulfolan gelöst und 15 h unter Stickstoff bei 80°C gerührt. Nach Zugabe von 6n-Salzsäure wurde einmal mit Essigester extrahiert, die wäßrige Phase mit Sodalösung alkalisiert und mehrfach mit Essigester extrahiert. Die organische Phase wurde getrocknet und tropfenweise mit einer Lösung von 0,7 g L(+)-Weinsäure in Aceton versetzt. Das

ausgefallene Hydrogentartrat wurde abgesaugt, mit Essigester gewaschen und getrocknet.

Ausbeute: 1,9 g (64%)

Fp.: 119°C

reine iso-Form

Beispiele 11—14

Alkylierung von 10-Bromisosandwicin

11) $N_b$-Methyl-10-bromisosandwiciniumjodid

Abweichend von Beispiel la wurde die Mutterlauge eingeengt und in wenig Methylenchlorid aufgenommen, wobei weiteres Produkt auskristallisierte.

Ausbeute: 97%

Fp.: 200°C

reine iso-Form

12a) $N_b$-n-Propyl-10-bromisosandwiciniumjodid

Die Darstellung erfolgt analog Beispiel 1a.

Ausbeute: 87%

Fp.: 265°C (dec)

reine iso-Form

12b) $N_b$-n-Propyl-10-bromisosandwiciniumhydrogentartrat

12 g $N_b$-n-Propyl-10-bromisosandwiciniumjodid wurden mit 300 ml verdünnter Sodalösung aufgenommen, dreimal mit Methylenchlorid extrahiert, die organische Phase getrocknet, stark eingeengt und mit 600 ml Essigester aufgenommen. Zu dieser Lösung tropfte man eine konzentrierte Lösung von 3,1 g L(+)-Weinsäure in Aceton. Das ausgefällte Hydrogentartrat wurde abfiltriert und mit Essigester gewaschen.

Ausbeute: 81%

Fp.: 149—151°C

reine iso-Form

12c) $N_b$-n-Propyl-10-bromisosandwicinium-dihydrogencitrat

1,2 g $N_b$-n-Propyl-10-bromisosandwiciniumjodid wurden mit 50 ml verdünnter Sodalösung aufgenommen und zweimal mit je 50 ml Essigester extrahiert. Zu dieser Lösung wurde eine mit etwas Essigester verdünnte Lösung von 0,44 g Citronensäure-1-hydrat in 3 ml Aceton gegeben und bis zur Trockene eingedampft.

Ausbeute: 1,3 g (100%)

Fp.: 125°C

n: iso ca. 1:4

13) $N_b$-n-Hexyl-10-bromisosandwiciniumjodid

Abweichend von Beispiel la wurde die gesamte Reaktionslösung eingeengt und in wenig Methylenchlorid aufgenommen, wobei das Produkt ausgefällt wurde.

Ausbeute: 90%

Fp.: 245—246°C

n: iso ca. 1:3

14) $N_b$-(2-Morpholinoäthyl)-10-bromisosandwicinium-chlorid

1,5 g 10-Bromisosandwicin und 0,8 g N-(2-Chloräthylmorpholin) wurden in 12 ml Äthanol gelöst und 14 Stunden unter Rückfluß erhitzt. Das ausgefallene Produkt wurde abgesaugt, mit Methylenchlorid gewaschen und getrocknet.

Ausbeute: 1,5 g (73%)

Fp.: 257°C

reine iso-Form

Beispiel 15

Tabletten, enthaltend $N_b$-Propyl-10-bromsanwicinium-hydrogentartrat

Zusammensetzung:

| | | |
|---|---|---|
| Wirkstoff | 15 | Teile |
| Lactose . $H_2O$ | 105 | „ |
| Maisstärke | 58 | „ |
| Aerosil 200®[1] | 0,5 | „ |
| Kollidon 25®[2] | 10 | „ |
| Aerosil 200® | 0,2 | „ |
| Magnesiumstearat | 1,3 | „ |
| Total | 190 | Teile |

[1]Hochdisperses Siliziumdioxid 200 qm/g (Degussa)

[2]Polyvinyl pyrrolidon mit mittleren Molekulargewicht von 25000

Herstellungvorschrift:

Der Wirkstoff wird mit Lactose, Maisstärke und Aerosil gründlich vermischt. Die entstandene Mischung wird mit einer 40% igen wäßrigen Lösung von Kollidon 25 im Diosna-Pharmamischer durchfeuchtet undgranuliert. Die feuchte Masse wird durch ein 2 mm-sieb passiert, bei 40°C auf Horden getrocknet und anschließend durch ein 1,6 mm-Sieb passiert. Nach dem Vermischen des Granulates mit Aerosil und Magnesiumstearat im Kubusmischer werden damit Tabletten vom Gesamtgewicht 190 mg gepreßt, so daß jede Tablette 15 mg Wirkstoff enthält.

Beispiel 16

Kapseln, enthaltend $N_b$-Propyl-10-bromsandwicinium-hydrogentartrat

Zusammensetzung:

| | | |
|---|---|---|
| Wirkstoff | 15 | Teile |
| Lactose, granuliert | 69,5 | „ |
| STA-Rx 1500®[3] | 10 | „ |
| Aerosil 200®[1] | 0,5 | „ |
| Magnesiumstearat | 1,0 | „ |
| Total | 96 | Teile |

[1]Hochdisperses Siliziumdioxid 200 qm/g (Degussa)

[3]Im quellvermögen reduzierte Maisstärke. Warenzeichen der Firma A. E. Staly Mfg.

Herstellungsvorschrift:

In einen geeigneten Mischer wird der Wirkstoff mit STA-Rx 1500 vermischt. Man fügt die granulierte Lactose (Tablettose®) hinzu und vermischt. Aerosil 200 und Magnesiumstearat werden durch ein 0,2 mm-Sieb passiert und nacheinander mit dem Ansatz vermischt.

Auf einer automatisch arbeitenden Kapselfüll- und -schließmaschine wird die Pulvermasse in Hartgelatinekapseln der Größe 4 abgefüllt. Jede Kapsel enthält im Mittel 96 mg Pulvermischung, entsprechend 15 mg Wirksubstanz.

Beispiel 17
Tabletten, enthaltend $N_b$-Methyl-10-bromsandwicinium-hydrogentartrat

Zusammensetzung:

| | | |
|---|---|---|
| Wirkstoff | 15 | Teile |
| Lactose . $H_2O$ | 105 | " |
| Maisstärke | 58 | " |
| Aerosil 200®[1)] | 0,5 | " |
| Kollidon 25®[2)] | 10 | " |
| Aerosil 200® | 0,2 | " |
| Magnesiumstearat | 1,3 | " |
| Total | 190 | Teile |

[1)]Hochdisperses Siliziumdioxid 200 qm/g (Degussa)

[2)]Polyvinylpyrrolidon mit mittlerem Molekulargewicht von 25000

Herstellungsvorschrift:
Der Wirkstoff wird mit Lactose, Maisstärke und Aerosil gründlich vermischt. Die entstandene Mischung wird mit einer 40% igen wäßrigen Lösung von Kollidon 25 im Diosna-Pharmamischer durchfeuchtet und granuliert. Die feuchte Masse wird durch ein 2 mm-Sieb passiert, bei 40°C auf Horden getrocknet und anschließend durch ein 1,6 mm-Sieb passiert. Nach dem Vermischen des Granulates mit Aerosil und Magnesiumstearat im Kubusmischer werden damit Tabletten vom Gesamtgewicht 190 mg gepreßt, so daß jede Tablette 15 mg Wirkstoff enthält.

Beispiel 18
Kapseln, enthaltend $N_b$-Methyl-10-bromsandwicinium-hydrogentartrat

Zusammensetzung:

| | | |
|---|---|---|
| Wirkstoff | 15 | Teile |
| Lactose, granuliert | 69,5 | " |
| STA-Rx 1500®[3)] | 10 | " |
| Aerosil 200®[1)] | 0,5 | " |
| Magnesiumstearat | 1,0 | " |
| Total | 96 | Teile |

[1)]Hochdisperses Siliziumdioxid 200 qm/g (Degussa)

[3)]Im Quellvermögen reduzierte Maisstärke. Warenzeichen der Firma A. E. Staly Mfg.

Herstellungsvorschrift:
In einen geeigneten Mischer wird der Wirkstoff mitSTA-Rx 1500 vermischt. Man fügt die granulierte Lactose (Tablettose®) hinzu und vermischt. Aerosil 200 und Magnesiumstearat weden durch ein 0,2 mm-sieb passiert und nacheinander mit dem Ansatz vermischt.

Auf einer automatisch arbeitenden Kapsulfüll- und -schließmaschine wird die Pulvermasse in Hartgelatine-kapseln der Größe 4 abgefüllt. Jede Kapsel enthält im Mittel 96 mg Pulvermischung, entsprechend 15 mg Wirksubstanz.

**Patentansprüche für die Vertragsstaaten: BE CH DE FR GB IT LI LU NL SE**

1. $N_b$-quartäre 10-Bromsandwicin- und 10-Bromisosandwicin-Derivate der allgemeinen Formel I

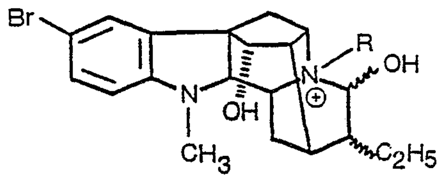

$A^{\ominus}$ (I)

in der

A das Anion einer anorganischen oder organischen Säure und

R ein bis zu 10 Kohlenstoffatome und gegebenenfalls Halogen oder Sauerstoff oder Stickstoff oder Sauerstoff und Stickstoff enthaltender Rest ist,

wobei

a) R ein Rest der allgemeinen Formel IIa

$$-(CH_2)_n-\underset{\displaystyle Y}{\underset{|}{CH}}-CH_2-X \qquad \text{(IIa)}$$

oder

b) R ein Rest der allgemeinen Formel IIb

$$-CH_2-\underset{\displaystyle OH}{\underset{|}{CH}}-CH_2-X \qquad \text{(IIb)}$$

ist, in denen

n den Wert 0 oder 1 annehmen kann

X Wasserstoff,
- gerad- oder verzweigtkettiges Alkyl,
- gegebenenfalls substituiertes Phenyl,
- Hydroxy,
- Dialkylamino,
- Pyrrolidino,
- Piperidino oder
- Morpholino und

Y Wasserstoff oder Methyl bedeuten oder

c) R ein Methyl-,
- Allyl-,
- Benzyl-,
- 4-Fluorbenzyl-,
- 4-Methoxybenzyl- oder
- 2-Hydroxy-2-phenyl-äthyl-Rest

ist.

2. Derivate nach Anspruch 1, dadurch gekennzeichnet, daß R ein Äthyl-, Propyl-, Butyl-, 3-Methylbutyl, Hexyl-, Decyl, 2-Hydroxy-äthyl-, 2-Diäthylamino-äthyl-, 3-Diäthylamino-propyl-, 2-(1-Pyrrolidinyl)-äthyl-, 2-(1-Piperidinyl)-äthyl-, 2-(4-Morpholinyl)-äthyl- oder 2-Hydroxy-3-(1-piperidinyl)-propyl-Rest ist.

3. Derivate nach Anspruch 1 oder 2, dadurch gekennzeichnet, daß A das Anion einer pharmakologisch annehmbaren Säure ist.

4. Derivate nach Anspruch 3, dadurch gekennzeichnet, daß A das Anion der Weinsäure ist.

5. Ringoffene Aldehydbasen der Formel IV

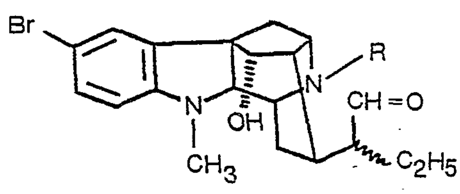

(IV)

in der R die im Anspruch 1 oder 2 genannte Bedeutung hat.

6. Verfahren zur Herstellung der Derivate nach einem der Anspruch 1 bis 4, wobei R die in Anspruch 1 für die Alternativen a) und b) genannte Bedeutung hat, dadurch gekennzeichnet, daß man 10-Bromsandwicin der Formel IIIn

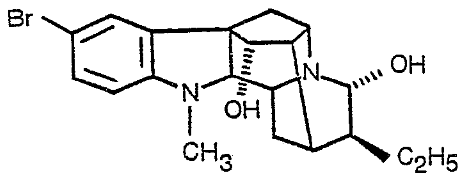

(III-n)

oder 10-Bromisosanwicin der Formel IIIi

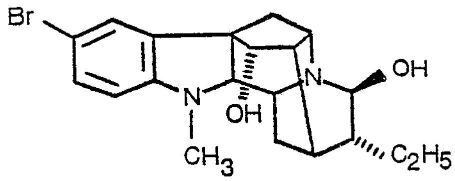

(III-i)

mit einer bis zu 10 Kohlenstoffatome enthaltenden Verbindung der Formeln Va oder Vb

$$Z{-}(CH_2)_n{-}\underset{\displaystyle Y}{\underset{|}{CH}}{-}CH_2{-}X \qquad (Va)$$

$$Z{-}CH_2{-}\underset{\displaystyle OH}{\underset{|}{CH}}{-}CH_2{-}X \qquad (Vb)$$

in denen, n, X und Y die in Anspruch 1 genannte Bedeutung haben und Z Chlor, Brom, Jod oder die Tosylgruppe bedeutet, umsetzt und gewünschtenfalls anschließend die erhaltenen quartären Salze durch Behandeln mit Alkalien in die ringoffenen Aldehydbasen der Formel IV, in der R die vorstehend genannte Bedeutung hat, umwandelt und diese durch Umsetzen mit einer Säure HA, in der A die in einem der Ansprüche 1, 3 oder 4 genannte Bedeutung hat, in das quartäre Derivat der Formel I, in der R die vorstehend und A die in einem der Ansprüche 1, 3 oder 4 genannte Bedeutung haben, überführt.

7. Verfahren zur Herstellung der Derivate nach einem der Ansprüche 1 bis 4, wobei R die in Anspruch 1 für die Alternative b) genannte Bedeutung hat, dadurch gekennzeichnet, daß man 10-Bromsandwicin der Formel IIIn oder 10-Bromisosandwicin der Formel IIIi mit einer bis zu 10 Kohlenstoffatome enthaltenden Verbindung der Formel Vb'

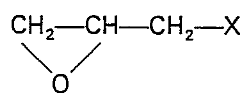

(Vb')

in der X die im Anspruch 1 genannte Bedeutung hat, umsetzt und anschließend die erhaltene ringoffene Aldehydbase der Formel IV, in der R die vorstehend genannte Bedeutung hat, durch Umsetzen mit einer Säure HA, in der A die in einem der Ansprüche 1, 3 oder 4 genannte Bedeutung hat, in das quartäre Derivat der Formel I, in der R die vorstehend und A die in einem der Ansprüche 1, 3 oder 4 genannte Bedeutung haben, überführt.

8. Verfahren zur Herstellung der Derivate nach einem der Ansprüche 1 bis 4, wobei R die in Anspruch 1 für die Alternative c) genannte Bedeutung, ausgenommen die Bedeutung 2-Hydroxy-2-phenyl-äthyl, hat, dadurch gekennzeichnet, daß man 10-Bromsandwicin der Formel IIIn oder 10-Bromisosandwicin der Formel IIIi mit

Methyl-,
Allyl-,
Benzyl-,
4-Fluorbenzyl-,
4-Methoxybenzyl-Chlorid, Bromid, Jodid, oder Tosylat

umsetzt und gewünschtenfalls anschließend die erhaltenen quartären Salze durch Behandeln mit Alkalien in die ringoffenen Aldehydbasen der Formel IV, in der R die vorstehend genannte Bedeutung hat, umwandelt und diese durch Umsetzen mit einer Säure HA, in der A die in einem der Ansprüche 1, 3 oder 4 genannte Bedeutung hat, in das quartäre Derivat der Formel I, in der R die vorstehend und A die in einem der Ansprüche 1, 3 oder 4 genannte Bedeutung haben, überführt.

9. Verfahren zur Herstellung der Derivate nach einem der Ansprüche 1 bis 4, wobei R 2-Hydroxy-2-phenyl-äthyl bedeutet dadurch gekennzeichnet, daß man 10-Bromsandwicin der Formel IIIn oder 10-Bromisosandwicin der Formel IIIi mit Epoxystyrol umsetzt und anschließend die erhaltene ringoffene Aldehydbase der Formel IV, in der R 2-Hydroxy-2-phenyl-äthyl bedeutet, durch Umsetzen mit einer Säure HA, in der A die in einem der Ansprüche 1, 3 oder 4 genannte Bedeutung hat, in das quartäre

Derivat der Formel I, in der R 2-Hydroxy-2-phenyl-äthyl bedeutet und A die in einem der Ansprüche 1, 3 oder 4 genannte Bedeutung hat, überführt.

10. Arzneimittel enthaltend ein Derivat gemäß Anspruch 3 oder 4.

**Patentansprüche für den Vertragsstaat: AT**

1. Verfahren zur Herstellung von $N_b$-quartären 10-Bromsandwicin- und 10-Bromisosandwicin-Derivaten der allgemeinen Formel I

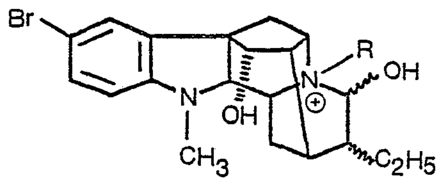

$A^{\ominus}$ (I)

in der

A das Anion einer anorganischen oder organischen Säure und

R ein bis zu 10 Kohlenstoffatome und gegebenenfalls Halogen oder Sauerstoff oder Stickstoff oder Sauerstoff und Stickstoff enthaltender Rest ist,

wobei

a) R ein Rest der allgemeinen Formel IIa

$$-(CH_2)_n-\underset{\displaystyle Y}{\underset{|}{C}H}-CH_2-X \qquad (IIa)$$

oder

b) R ein Rest der allgemeinen Formel IIb

$$-CH_2-\underset{\displaystyle OH}{\underset{|}{C}H}-CH_2-X \qquad (IIb)$$

ist, in denen

n den Wert 0 oder 1 annehmen kann

X Wasserstoff,
gerad- oder verzweigtkettiges Alkyl,
gegebenenfalls substituiertes Phenyl,
Hydroxy,
Dialkylamino,
Pyrrolidino,
Piperidino oder
Morpholino und

Y Wasserstoff oder Methyl bedeuten,

dadurch gekennzeichnet, daß man 10-Bromsandwicin der Formel IIIn

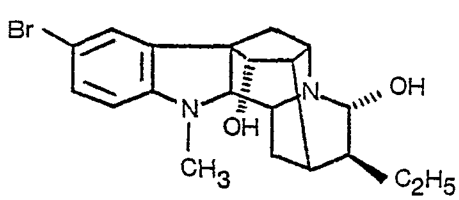

(III-n)

oder 10-Bromisosandwicin der Formel IIIi

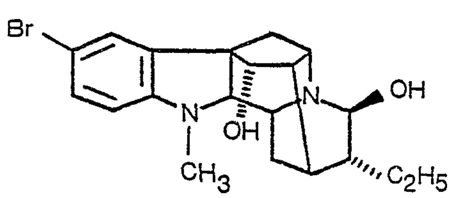

(III-i)

mit einer bis zu 10 Kohlenstoffatome enthaltenden Verbindung der Formeln Va oder Vb

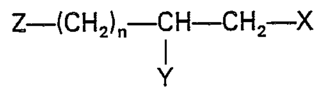
(Va)

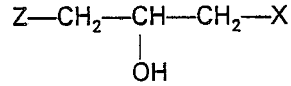
(Vb)

in denen n, X und Y die vorstehend genannte Bedeutung haben und Z Chlor, Brom, Jod oder dieTosylgruppe bedeutet, umsetzt und gewünschtenfalls anschließend die erhaltenen quartären Salze durch Behandeln mit Alkalien in die ringoffenen Aldehydbasen der Formel IV

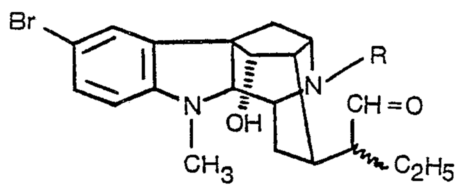
(IV)

in der R die vorstehend genannte Bedeutung hat, umwandelt und diese durch Umsetzen mit einer Säure HA, in der A die vorstehend genannte Bedeutung hat, in das quartäre Derivat der Formel I, in der R und A die vorstehend genannte Bedeutung haben, überführt.

2. Verfahren zur Herstellung von $N_b$-quartären 10-Bromsandwicin- und 10-Bromisosandwicin-Derivaten der allgemeinen Formel I, in der

A das Anion einer anorganischen oder organischen Säure und
R ein bis zu 10 Kohlenstoffatome und gegebenenfalls
Halogen oder Sauerstoff oder Stickstoff oder
Sauerstoff und Stickstoff enthaltender Rest ist, wobei R ein Rest der allgemeinen Formel IIb

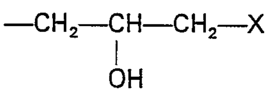
(IIb)

ist, in der X Wasserstoff,
gerad- oder verzweigtkettiges Alkyl,
gegebenenfalls substituiertes Phenyl,
Hydroxy,
Dialkylamino,
Pyrrolidino,
Piperidino oder
Morpholino bedeutet,

dadurch gekennzeichnet, daß man 10-Bromsandwicin der Formel IIIn oder 10-Bromisosandwicin der Formel IIIi mit einer bis zu 10 Kohlenstoffatome enthaltenden Verbindung der Formel Vb'

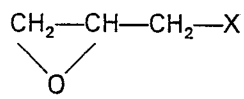
(Vb')

in der X die vorstehend genannte Bedeutung hat, umsetzt und anschließend die erhaltene ringoffene Aldehydbase der Formel IV, in der R die vorstehend genannte Bedeutung hat, durch Umsetzen mit einer Säure HA, in der A die vorstehend genannte Bedeutung hat, in das quartäre Derivat der Formel I, in der R und A die vorstehend genannte Bedeutung haben, überführt.

3. Verfahren zur Herstellung von $N_b$-quartären 10-Bromsandwicin- und 10-Bromisosandwicin-Derivaten der allgemeinen Formel I, in der A das Anion einer anorganischen oder organischen Säure und R ein

Methyl-,
Allyl-,
Benzyl-,
4-Fluorbenzyl- oder
4-Methoxybenzyl-Rest ist,

dadurch gekennzeichnet, daß man 10-Bromsandwicin der Formel IIIn oder 10-Bromisosandwicin der Formel IIIi mit einem

Methyl-,
Allyl-,
Benzyl-,
4-Fluorbenzyl-,

4-Methoxybenzyl-Chlorid, Bromid, Jodid, oder Tosylat umsetzt und gewunschtenfalls anschließend die erhaltenen quartären Salze durch Behandeln mit Alkalien in die ringoffenen Aldehydbasen der Formel IV, in der R die vorstehend genannte Bedeutung hat, umwandelt und diese durch Umsetzen mit einer Säure HA, in der A die vorstehend genannte Bedeutung hat, in das quartäre Derivat der Formel I, in der R und A die vorstehend genannte Bedeutung haben, überführt.

4. Verfahren zur Herstellung von $N_b$-quartären 10-Bromsandwicin- und 10-Bromisosandwicin-Derivaten der allgemeinen Formel I, in der A das Anion einer anorganischen oder organischen Säure und R ein 2-Hydroxy-2-phenyl-äthyl-Rest ist, dadurch gekennzeichnet, daß man 10-Bromsandwicin der Formel IIIn oder 10-Bromisosandwicin der Formel IIIi, mit Epoxystyrol umsetzt und anschließend die erhaltene ringoffene Aldehydbase der Formel IV, in der R die vorstehend genannte Bedeutung hat, durch Umsetzen mit einer Säure HA, in der A die vorstehend genannte Bedeutung hat, in das quartäre Derivat der Formel I, in der R und A die vorstehend genannte Bedeutung haben, überführt.

**Claims for the Contracting States: BE CH DE FR GB IT LI LU NL SE**

1. $N_b$-quaternary 10-bromosandwicine derivatives and 10-bromoisosandwicine derivatives of the general formula I

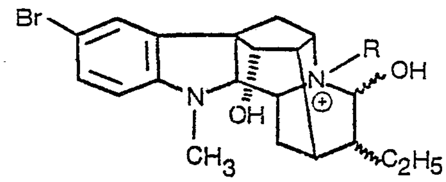

$A^{\ominus}$ (I)

in which

A is the anion of an inorganic or organic acid and

R is a radical containing up to 10 carbon atoms and optionally containing halogen or oxygen or nitrogen or oxygen and nitrogen,

where

a) R is a radical of the general formula IIa

$$—(CH_2)_n—\underset{\underset{Y}{|}}{CH}—CH_2—X \qquad (IIa)$$

or

b) R is a radical of the general formula IIb

$$—CH_2—\underset{\underset{OH}{|}}{CH}—CH_2—X \qquad (IIb)$$

in which

n can assume the value 0 or 1,

X signifies hydrogen,
- straight-chain or branched alkyl,
- optionally substituted phenyl,
- hydroxy,
- dialkylamino,
- pyrrolidino,
- piperidino or
- morpholino and

Y signifies hydrogen or methyl

or

c) R is a methyl-,
- allyl-,
- benzyl-,
- 4-fluorobenzyl-,
- 4-methoxybenzyl- or
- 2-hydroxy-2-phenyl-ethyl- radical.

2. Derivatives according to Claim 1, characterized in that R is an ethyl, propyl, butyl, 3-methylbutyl, hexyl, decyl, 2-hydroxy-ethyl, 2-diethylamino-ethyl, 3-diethylamino-propyl, 2-(1-pyrrolidinyl)-ethyl, 2-(1-piperidinyl)-ethyl, 2-(4-morpholinyl)-ethyl or 2-hydroxy-3-(1-piperidinyl)-propyl radical.

3. Derivatives according to Claim 1 or 2, characterized in that A is the anion of a pharmacologically acceptable acid.

4. Derivatives according to Claim 3, characterized in that A is the anion of tartaric acid.

5. Ring-opened aldehyde bases of the formula IV

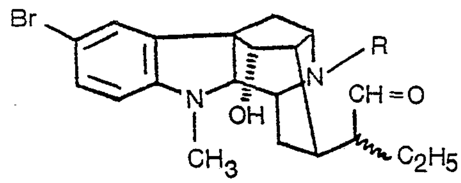

(IV)

in which R has the meaning indicated in Claim 1 or 2.

6. Process for the preparation of the derivatives according to one of Claims 1 to 4, in which R has the meaning indicated in Claim 1 for the alternatives a) and b), characterized in that 10-bromosandwicine of the formula IIIn

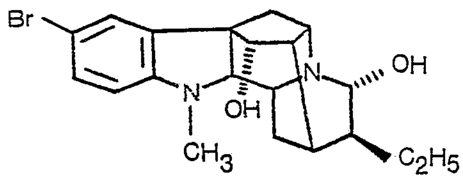

(III-n)

or 10-bromoisosandwicine of the formula IIIi

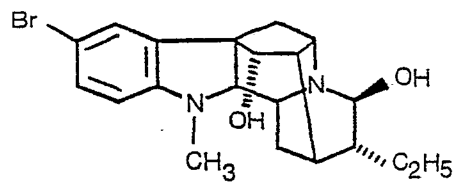

(III-i)

is reacted with a compound, containing up to 10 carbon atoms, of the formula Va or Vb

$$Z{-}(CH_2)_n{-}\underset{\displaystyle Y}{\underset{|}{CH}}{-}CH_2{-}X \qquad \text{(Va)}$$

$$Z{-}CH_2{-}\underset{\displaystyle OH}{\underset{|}{CH}}{-}CH_2{-}X \qquad \text{(Vb)}$$

in which n, X and Y have the meaning indicated in Claim 1 and Z signifies chlorine, bromine, iodine or the tosyl group, and, if desired, the resulting quaternary salts are then converted into ring-opened aldehyde bases of the formula IV in which R has the meaning indicated above by treatment with alkalis, and these bases are converted into the quaternary derivative of the formula I, in which R has the above-mentioned meaning and A has the meaning indicated in one of Claims 1, 3 or 4, by reaction with an acid HA, in which A has the meaning indicated in one of Claims 1, 3 or 4.

7. Process for the preparation of the derivatives according to one of Claims 1 to 4, in which R has the meaning indicated in Claim 1 for alternative b), characterized in that 10-bromosandwicine of the formula IIIn or 10-bromoisosandwicine of the formula IIIi is reacted with a compound of the formula Vb′, containing up to 10 carbon atoms

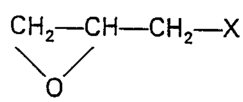

(Vb′)

in which X has the meaning indicated in Claim 1, and subsequently the obtained ring-opened aldehyde base of the formula IV, in which R has the above-mentioned meaning, is converted into the quaternary derivative of the formula I, in which R has the above-mentioned meaning and A has the meaning indicated in one of Claims 1, 3 or 4, by reaction with an acid HA, in which A has the meaning indicated in one of Claims 1, 3 or 4.

8. Process for the preparation of the derivatives according to one of Claims 1 to 4, in which R has the meaning indicated in Claim 1 for alternative c), except for the meaning 2-hydroxy-2-phenyl-ethyl,

characterized in that 10-bromosandwicine of the formula IIIn, or 10-bromoisosandwicine of the formula IIIi is reacted with

methyl-
allyl-
benzyl-
4-fluorobenzyl-
4-methoxybenzyl-chloride, bromide, iodide or tosylate

and optionally, subsequently, the obtained quaternary salts are converted by treatment with alkalis into the ring-opened aldehyde bases of formula IV, in which R has the above-mentioned meaning, and these are converted into the quaternary derivative of formula I, in which R has the above-mentioned meaning and A has the meaning indicated in one of Claims 1, 3 or 4, by reaction with an acid HA, in which A has the meaning indicated in one of Claims 1, 3 or 4.

9. Process for the preparation of the derivatives according to one of Claims 1 to 4, in which R signifies 2-hydroxy-2-phenyl-ethyl, characterized in that 10-bromosandwicine of the formula IIIn or 10-bromoisosandwicine of the formula IIIi is reacted with epoxystyrene and subsequently the obtained, ring-opened aldehyde base of formula IV, in which R signifies 2-hydroxy-2-phenyl-ethyl, is converted into the quaternary derivative of formula I, in which R signifies 2-hydroxy-2-phenyl-ethyl and A has the meaning indicated in one of Claims 1, 3 or 4, by reaction with an acid HA, in which A has the meaning indicated in one of Claims 1, 3 or 4.

10. Medicaments containing a derivative according to Claim 3 or 4.

**Claims for the Contracting State: AT**

1. Process for the preparation of $N_b$-quaternary 10-bromosandwicine derivatives and 10-bromoisosandwicine derivatives of the general formula I

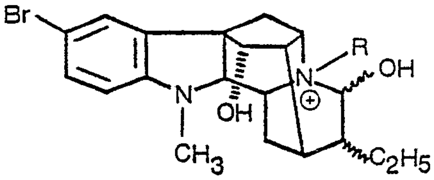

$A^{\ominus}$ (I)

in which

A is the anion of an inorganic or organic acid and

R is a radical containing up to 10 carbon atoms and optionally containing halogen or oxygen or nitrogen or oxygen and nitrogen,

where

a) R is a radical of the general formula IIa

$$-(CH_2)_n-\underset{\displaystyle Y}{\underset{|}{CH}}-CH_2-X \qquad \text{(IIa)}$$

or

b) R is a radical of the general formula IIb

$$-CH_2-\underset{\displaystyle OH}{\underset{|}{CH}}-CH_2-X \qquad \text{(IIb)}$$

in which

n can assume the value 0 or 1,

X signifies hydrogen,
straight-chain or branched alkyl,
optionally substituted phenyl,
hydroxy,
dialkylamino,
pyrrolidino,
piperidino or
morpholino and

Y signifies hydrogen or methyl,

characterized in that 10-bromosandwicine of the formula IIIn

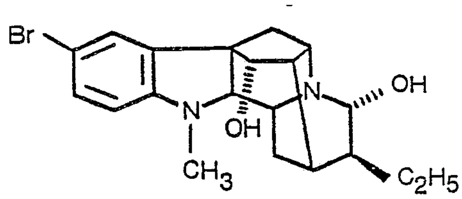

(III-n)

or 10-bromoisosandwicine of the formula IIIi

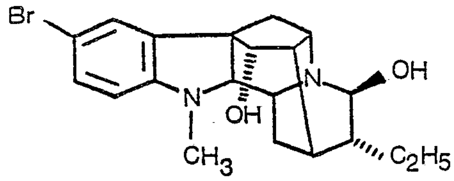

(III-i)

is reacted with a compound, containing up to 10 carbon atoms, of the formulae Va or Vb

$$Z-(CH_2)_n-\underset{\displaystyle Y}{\underset{|}{CH}}-CH_2-X \qquad (Va)$$

$$Z-CH_2-\underset{\displaystyle OH}{\underset{|}{CH}}-CH_2-X \qquad (Vb)$$

in which n, X and Y have the above-mentioned meaning and Z signifies chlorine, bromine, iodine or the tosyl group, and, if desired, the resulting quaternary salts are then converted into ring-opened aldehyde bases of the formula IV

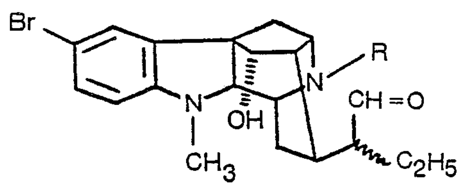

(IV)

in which R has the meaning indicated above by treatment with alkalis, and these bases are converted into the quaternary derivative of the formula I, in which R and A have the above-mentioned meaning, by reaction with an acid HA, in which A has the above-mentioned meaning.

2. Process for the preparation of $N_b$-quaternary 10-bromosandwicine derivatives and 10-bromoisosandwicine derivatives of the general formula I,
in which

A is the anion of an inorganic or organic acid and

R is a radical containing up to 10 carbon atoms and optionally containing halogen or oxygen or nitrogen or oxygen and nitrogen,

where R is a radical of the general formula IIb

$$-CH_2-\underset{\displaystyle OH}{\underset{|}{CH}}-CH_2-X \qquad (IIb)$$

in which X signifies hydrogen,
- straight-chain or branched alkyl,
- optionally substituted phenyl,
- hydroxy,
- dialkylamino,
- pyrrolidino,
- piperidino or
- morpholino,

characterized in that 10-bromosandwicine of the formula IIIn or 10-bromoisosandwicine of the formula IIIi is reacted with a compound of the formula Vb′, containing up to 10 carbon atoms

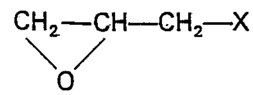

(Vb')

in which X has the above-mentioned meaning, and subsequently the obtained ring-opened aldehyde base of the formula IV, in which R has the above-mentioned meaning, is converted into the quaternary derivative of the formula I, in which R and A have the above-mentioned meaning, by reaction with an acid HA, in which A has the above-mentioned meaning.

3. Process for the preparation of $N_b$-quaternary derivatives of 10-bromosandwicine and 10-bromoisosandwicine of the general formula I, in which A is the anion of an inorganic or organic acid and R is a

methyl-,
allyl-,
benzyl-,
4-fluorobenzyl- or
4-methoxybenzyl- radical,

characterized in that 10-bromosandwicine of the formula IIIn, or 10-bromoisosandwicine of the formula IIIi is reacted with a

methyl-,
allyl-,
benzyl-,
4-fluorobenzyl-,
4-methoxybenzyl-chloride, bromide, iodide or tosylate

and optionally, subsequently, the obtained quaternary salts are converted by treatment with alkalis into the ring-opened aldehyde bases of formula IV, in which R has the above-mentioned meaning, and these are converted into the quaternary derivative of formula I, in which R and A have the above-mentioned meaning, through reaction with an acid HA, in which A has the above-mentioned meaning.

4. Process for the preparation of $N_b$-quaternary 10-bromosandwicine derivatives and 10-bromoisosandwicine derivatives of the general formula I, in which A is the anion of an inorganic or organic acid, and R is a 2-hydroxy-2-phenylethyl radical, characterized in that 10-bromosandwicine of the formula IIIn or 10-bromoisosandwicine of the formula IIIi is reacted with epoxystyrene and subsequently the obtained, ring-opened aldehyde base of formula IV, in which R has the above-mentioned meaning is converted into the quaternary derivative of formula I, in which R and A have the above-mentioned meaning, by reaction with an acid HA, in which A has the above-mentioned meaning.

**Revendications pour les Etats contractants: BE CH DE FR GB IT LI LU NL SE**

1. Dérivés $N_b$-quaternaires de 10-bromo-sandwicine et de 10-bromo-isosandwicine, de formule générale I

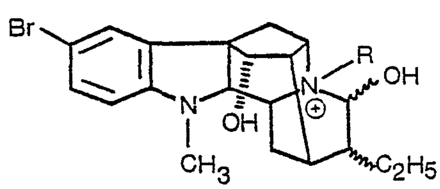

$A^{\ominus}$ (I)

dans laquelle A est l'anion d'un acide inorganique ou organique et R est un radical contenant jusqu'à 10 atomes de carbone et éventuellement un halogène ou de l'oxygène ou de l'azote ou de l'oxygène et de l'azote,

où

a) R est un radical de formule générale IIa

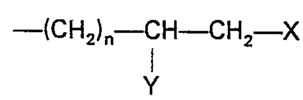

(IIa)

ou

b) R est un radical de formule générale IIb

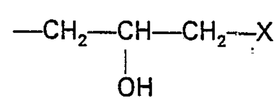

(IIb)

dans lesquelles n peut prendre la valeur 0 ou 1; X est l'hydrogène, un radical alkyle à chaîne droite ou ramifiée, phényle éventuellement substitué, hydroxy, dialkylamino, pyrrolidino, pipéridino ou morpholino; et Y est l'hydrogène ou le radical méthyle; ou bien

c) R est un radical méthyle, allyle, benzyle, 4-fluorobenzyle, 4-méthoxybenzyle ou 2-hydroxy-2-phényle-éthyle.

2. Dérivés selon la revendication 1, caractérisés en ce que R est un radical éthyle, propyle, butyle, 3-méthylbutyle, hexyle, décyle, 2-hydroxy-éthyle, 2-diéthylaminoéthyle, 3-diéthylamino-propyle, 2-(1-pyrrolidinyl)-éthyl, 2-(1-pipéridinyl)-éthyle, 2-(4-morpholinyl)-éthyle ou 2-hydroxy-3-(1-pipéridinyl)-propyle.

3. Dérivés selon la revendication 1 ou 2, caractérisés en ce que A est l'anion d'un acide pharmacologiquement acceptable.

4. Dérivés selon la revendication 3, caractérisés en ce que A est l'anion de l'acide tartrique.

5. Aldéhydes-bases à cycle ouvert de formule IV

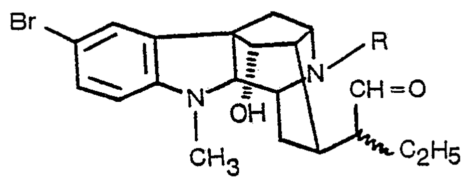

(IV)

dans laquelle R a la signification donnée dans la revendication 1 ou 2.

6. Procédé pour la préparation des dérivés selon l'une des revendications 1 à 4, où R a la signification donnée dans la revendication 1 aux points a) et b), caractérisé en ce que l'on fait réagir la 10-bromosandwicine de formule IIIn

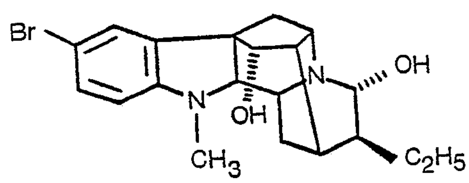

(III-n)

ou la 10-bromo-isosandwicine de formule IIIi

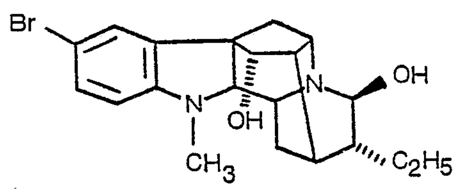

(III-i)

sur un composé contenant jusqu'a 10 atomes de carbone, de formules Va ou Vb

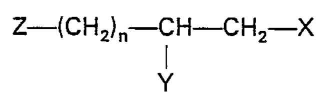

(Va)

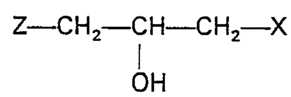

(Vb)

dans lesquelles n, X et y ont la signification donnée dans la revendication 1 et Z est le chlore, le brome, l'iode ou le groupe tosyle, et, si on le souhaite, on transforme ensuite les sels quaternaires obtenus par traitement avec des alcalis en les aldéhydes-bases à cycle ouvert de formule IV, dans laquelle R est la signification donnée ci-dessus, puis l'on convertit ces dernières par réaction avec un acide HA, où A la signification donnée dans l'une des revendications 1, 3 ou 4, en le dérivé quaternaire de formule I où R a la signification donnée ci-dessus et A la signification donnée dans l'une des revendications 1, 3 ou 4.

7. Procédé pour la préparation de dérivés selon l'une des revendications 1 à 4, où R a la signification donnée dans la revendication 1 au point b), caractérisé en ce que l'on fait réagir de la 10-bromo-sandwicine de formule IIIn ou de la 10-bromo-isosandwicine de formule IIIi sur un composé contenant jusqu'à 10 atomes de carbone, de formule Vb'

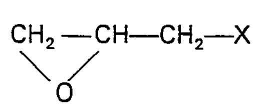

(Vb')

où X a la signification donnée dans la revendication 1, et l'on convertit ensuite l'aldéhyde-base à cycle ouvert obtenue, de formule IV, dans laquelle R a la signification donnée ci-dessus, et par réaction sur un acide HA où A a la signification donnée dans l'une des revendications 1, 3 ou 4, en le dérivé quaternaire de formule I où R a la signification donnée ci-dessus et A a la signification donnée dans l'une des revendications 1, 3 ou 4.

8. Procédé pour la préparation des dérivés selon l'une des revendications 1 à 4, où R a la signification donnée dans la revendication 1 ou point c), à l'exception de la signification 2-hydroxy-2-phényl-éthyle, caractérisé en ce qu'on fait réagir de la 10-bromo-sandwicine de formule IIIn ou de la 10-bromo-isosandwicine de formule IIIi, sur du chlorure, bromure, iodure ou toxylate de méthyle, allyle, benzyle, 4-fluorobenzyle, 4-méthoxybenzyle, et, si on le souhaite, on transforme ensuite les sels quaternaires obtenus, par traitement avec des alcalis, en les aldéhydes-bases à cycle ouvert de formule IV où R a la signification donnée ci-dessus, et l'on convertit ces dernières, par réaction avec un acide HA où A a la signification donnée dans l'une des revendications 1, 3 ou 4, en le dérivé quaternaire de formule I où R a la signification donnée ci-dessus et A la signification donnée dans l'une des revendications 1, 3 ou 4.

9. Procédé pour la préparation des dérivés selon l'une des revendications 1 à 4, où R est le radical 2-hydroxy-2-phényl-éthyle, caractérisé en ce qu'on fait réagir de la 10-bromo-sandwicine de formule IIIn ou de la 10-bromo-isosandwicine de formule IIIi sur de l'époxystyrène, puis l'on convertit l'aldéhyde-base à cycle ouvert obtenue de formule IV, où R est le radical 2-hydroxy-2-phényl-éthyle, par réaction avec un acide HA où A a la signification donnée dans l'une des revendications 1, 3 ou 4, en le dérivé quaternaire de formule I où R est le radical 2-hydroxy-2-phényl-éthyle et A a la signification donnée dans l'une des revendications 1, 3 ou 4.

10. Médicament contenant un dérivé selon la revendication 3 ou 4.

**Revendications pour les Etats contractants: AT**

1. Procédé pour la préparation de dérivés $N_b$-quaternaires de 10-bromo-sandwicine et de 10-bromo-isosandwicine de formule générale I

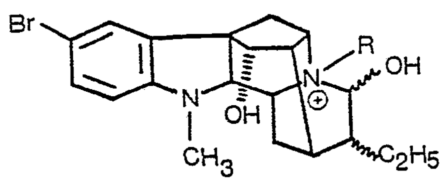 $A^{\ominus}$ (I)

où A est l'anion d'un acide inorganique ou organique et R est un radical contenant jusqu'à 10 atomes de carbone et éventuellement un halogène ou de l'oxygène ou de l'azote ou de l'oxygène et de l'azote, où

a) R est un radical de formule générale IIa

$$-(CH_2)_n-\underset{\substack{|\\ Y}}{CH}-CH_2-X \qquad \text{(IIa)}$$

ou

b) R est un radical de formule générale IIb

$$-CH_2-\underset{\substack{|\\ OH}}{CH}-CH_2-X \qquad \text{(IIb)}$$

dans lesquelles n peut prendre la valeur 0 ou 1; X est l'hydrogène, un radical alkyle à chaîne droite ou ramifiée, phényle éventuellement substitué, hydroxy, dialkylamino, pyrrolidino, pipéridino ou morpholino; et Y est l'hydrogène ou le radical méthyle; caractérisé en ce que l'on fait réagir de la 10-bromo-sandwicine de formule IIIn

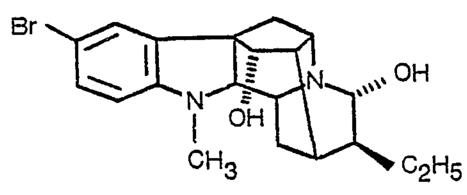 (III-n)

ou de la 10-bromo-isosandwicine de formule IIIi

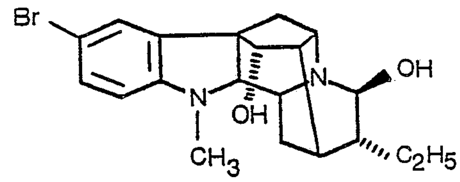

(III-i)

sur un composé contenant jusqu'à 10 atomes de carbone, de formule Va ou Vb

$$Z{-}(CH_2)_n{-}CH(Y){-}CH_2{-}X \qquad \text{(Va)}$$

$$Z{-}CH_2{-}CH(OH){-}CH_2{-}X \qquad \text{(Vb)}$$

dans lesquelles n, X et Y ont la signification donnée ci-dessus et Z est le chlore, le brome, l'iode ou le groupe tosyle, et, si on le souhaite, on transforme les sels quaternaires obtenus par traitement avec des alcalis, en les aldéhydes-bases à cycle ouvert de formule IV

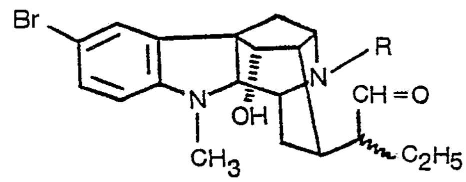

(IV)

dans laquelle R a la signification donnée ci-dessus, et l'on convertit ces dernières par réaction sur un acide HA, où A a la signification donnée ci-dessus, en le dérivé quaternaire de formule I où R et A ont la signification donnée ci-dessus.

2. Procédé pour la préparation de dérivés $N_b$-quaternaires de 10-bromo-sandwicine et de 10-bromo-isosandwicine de formule générale I, dans laquelle A est l'anion d'un acide inorganique ou organique et R est un radical contenant jusqu'à 10 atomes de carbone et éventuellement un halogène ou de l'oxygène ou de l'azote ou de l'oxygène et de l'azote, où R est un radical de formule générale IIb

$$-CH_2{-}CH(OH){-}CH_2{-}X \qquad \text{(IIb)}$$

dans laquelle X est l'hydrogène, un radical alkyle à chaîne droite ou ramifiée, phényle éventuellement substitué, hydroxy, dialkylamino, pyrrolidino, pipéridino ou morpholino, caractérisé en ce que l'on fait réagir de la 10-bromo-sandwicine de formule IIIn ou de la 10-bromo-isosandwicine de formule IIIi sur un composé contenant jusqu'à 10 atomes de carbone, de formule Vb'

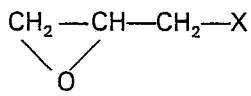

(Vb')

dans laquelle X a la signification donnée ci-dessus, puis l'on convertit l'aldéhyde-base à cycle ouvert obtenue, de formule IV dans laquelle R a la signification donnée ci-dessus, par réaction avec un acide HA où A a la signification donnée ci-dessus, en le dérivé quaternaire de formule I où R et A ont la signification donnée ci-dessus.

3. Procédé pour la préparation de dérivés $N_b$-quaternaires de 10-bromo-sandwicine et de 10-Bromo-isosandwicine de formule générale I, dans laquelle A est l'anion d'un acide inorganique ou organique et R est un radical méthyle, allyle, benzyle, 4-fluorobenzyle ou 4-méthoxybenzyle, caractérisé en ce qu'on fait réagir la 10-bromo-sandwicine de formule IIIn ou la 10-bromo-isosandwicine de formule IIIi sur un chlorure, bromure, iodure ou tosylate de méthyle, allyle, benzyle, 4-fluorobenzyle, 4-méthoxybenzyle, et si on le souhaite, on transforme ensuite les sels quaternaires obtenus par traitement avec des alcalis, en les aldéhydes-bases de formule IV dans laquelle R a la signification donnée ci-dessus, et l'on transforme ces dernières, par réaction avec un acide HA où A a la signification donnée ci-dessus, en le dérivé quaternaire de formule I dans laquelle R et A ont la signification donnée ci-dessus.

4. Procédé pour la préparation de dérivés $N_b$-quaternaires de 10-bromo-sandwicine et de 10-bromo-isosandwicine de formule générale I dans laquelle A est l'anion d'un acide inorganique ou organique et R est un radical 2-hydroxy-2-phényle-éthyle, caractérisé en ce qu'on fait réagir de la 10-bromo-sandwicine de formule IIIn ou de la 10-bromo-isosandwicine de formule IIIi sur de l'époxystyrène, puis l'on transforme l'aldéhyde-base à cycle ouvert obtenue, de formule IV dans laquelle R as la signification donnée ci-dessus, par réaction avec un acide HA où A a la signification donnée ci-dessus, en le dérivé quaternaire de formule I dans laquelle R et A ont la signification donnée ci-dessus.

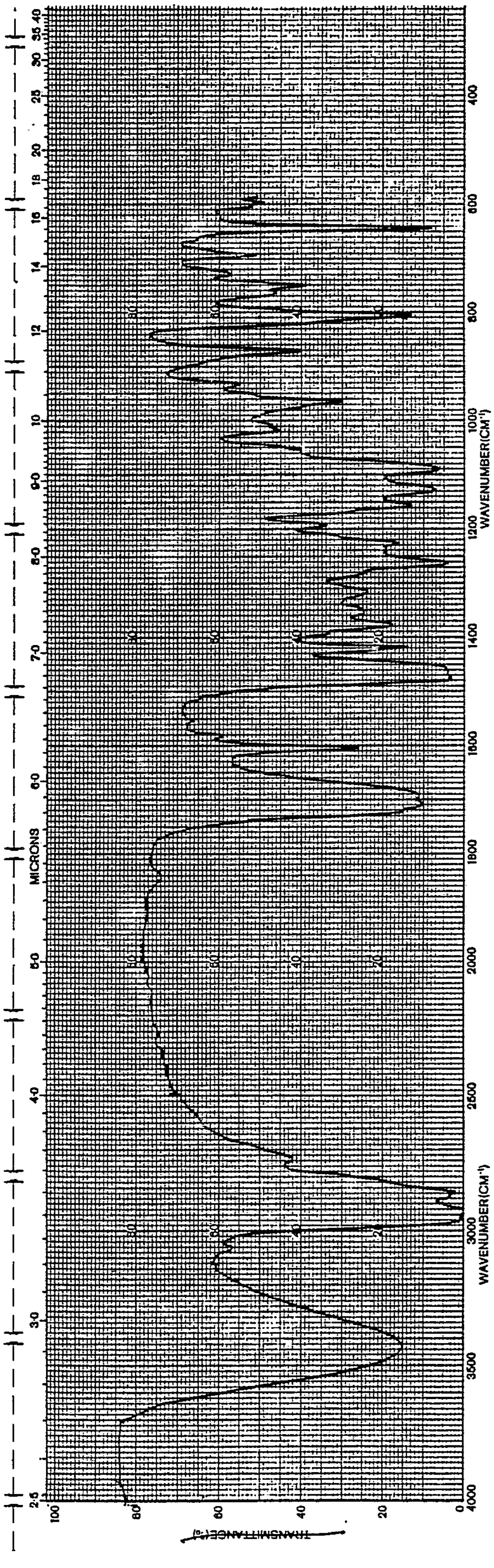


FIG.1

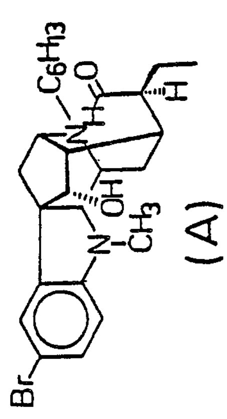

(A)

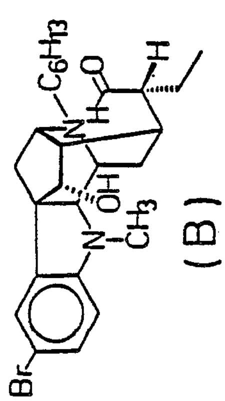

(B)

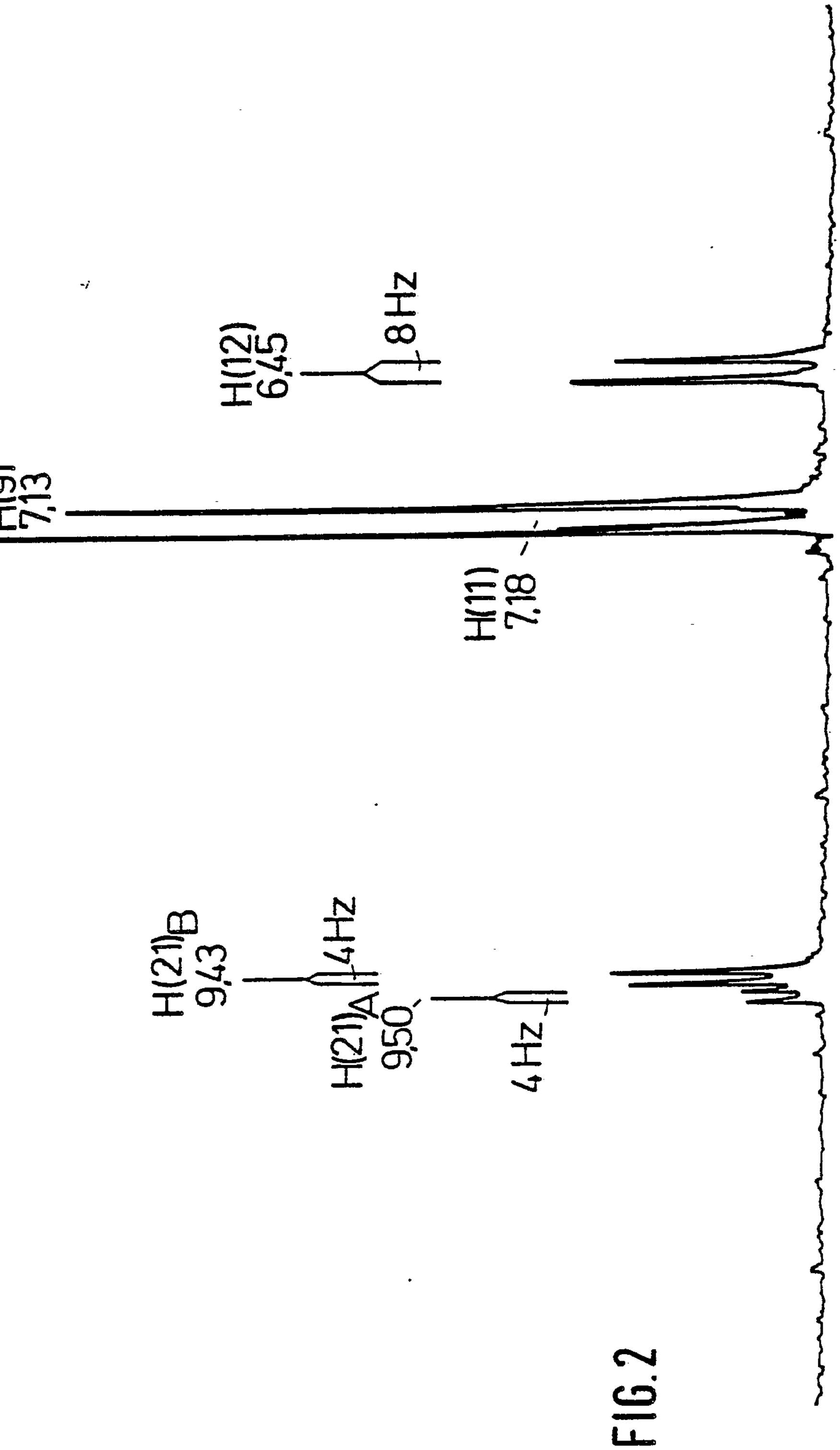


FIG. 2